# EUROPEAN PATENT APPLICATION

(11) **EP 4 353 708 A1**
(43) Date of publication of application: **17.04.2024**
(21) Application number: 22816117.0
(22) Date of filing: 31.05.2022
(51) Int. Cl.: C07C 51/43, B01D 9/02, C02F 1/42, C07C 51/25, C07C 57/055

(54) **METHOD FOR PRODUCING EASILY POLYMERIZABLE COMPOUND**

(30) Priority: 02.06.2021 JP 2021093030
(71) Applicant: Nippon Shokubai Co., Ltd., Osaka-shi, Osaka 541-0043 (JP)
(72) Inventor: MUKAE, Masashi, Himeji-shi, Hyogo 671-1282 (JP); TAKEMOTO, Yasutaka, Himeji-shi, Hyogo 671-1282 (JP); UENO, Takamasa, Himeji-shi, Hyogo 671-1282 (JP)
(74) Representative: Schön, Christoph
(86) International application number: PCT/JP2022/022153
(87) International publication number: WO 2022/255371

(57) **Abstract**

The present invention provides a method for efficiently obtaining a high-quality product. The present invention relates to a method for producing an easily polymerizable compound, the method including: mixing an easily polymerizable compound and a manganese-based polymerization inhibitor; adjusting a manganese concentration in an easily polymerizable compound-containing solution obtained through the mixing by bringing at least a portion of the easily polymerizable compound-containing solution into contact with a cation exchange resin; and crystallizing from an easily polymerizable compound-containing solution having a manganese concentration of 5 mass ppm or lower obtained through the adjusting.

## Description

### TECHNICAL FIELD

The present invention relates to methods for producing easily polymerizable compounds. Specifically, the present invention relates to a method for producing an easily polymerizable compound, a method for purifying an easily polymerizable compound, and an apparatus for purifying an easily polymerizable compound.

### BACKGROUND ART

Purification apparatuses have been widely used industrially to purify compounds that are used as raw materials for resins, for example. In many fields of the chemical industry, high-quality compounds with reduced impurities have been required, and for such compounds, various better purification apparatuses have been studied.

Industrially, many of crude compounds, which are compounds before purification, are purified through continuous purification processes. Disclosed is a method for producing acrylic acid including: collecting and crystallization purifying a gas containing acrylic acid obtained by catalytic vapor phase oxidation of a raw material gas; and returning acrylic acid obtained by decomposing a substance obtained by Michael addition of acrylic acid in a residual mother liquor to the collecting step, for example (see, for example, Patent Literature 1).

Here, particularly in production of easily polymerizable compounds such as acrylic acid, it is known that a polymerization inhibitor is added for the purpose of preventing polymerization during the purification (e.g., distillation). An example of a polymerization inhibitor is a polymerization inhibitor containing a transition metal component such as manganese or copper.

Patent Literature 2 discloses the following technique: to prevent a transition metal component derived from a polymerization inhibitor contained in a crude acrylic acid from deactivating an esterification reaction catalyst, for example, water is added to the crude acrylic acid, and the resulting solution is brought into contact with a cation exchange resin, whereby the transition metal component is efficiently removed.

Patent Literature 3 discloses the following technique: in obtaining a purified (meth)acrylic acid from a crude (meth)acrylic acid by repeating a crystallization operation in multiple times, the polymerization inhibitor concentration in a (meth)acrylic acid-containing solution to be subjected to a crystallizing in the first crystallization operation is adjusted so that the polymerization inhibitor concentration in a (meth)acrylic acid-containing solution to be subjected to the crystallizing in the final crystallization operation is equal to or higher than a predetermined concentration, whereby polymerization of (meth)acrylic acid can be easily prevented.

### CITATION LIST

### - Patent Literature

Patent Literature 1: JP 2007-182437 A
Patent Literature 2: JP 2005-187332 A
Patent Literature 3: JP 2014-65705 A

### SUMMARY OF INVENTION

### - Technical Problem

As described above, better methods for producing easily polymerizable compounds have been desired, and methods for efficiently obtaining high-quality products (easily polymerizable compounds) have been desired. The present invention has been made in view of the above-mentioned current state of the art, and aims to provide a method for efficiently obtaining a high-quality product.

### - Solution to Problem

The present inventors have investigated a method for efficiently obtaining a high-quality product, and focused on a polymerization inhibitor. In conventional production of easily polymerizable compounds, a polymerization inhibitor such as a manganese-based polymerization inhibitor has been added for the purpose of preventing polymerization in the purification. The present inventors found a problem in such production. Specifically, when an easily polymerizable compound-containing solution containing a manganese-based polymerization inhibitor is subjected to crystallization, impurities and manganese in the easily polymerizable compound-containing solution form a complex as a precipitate under crystallization conditions, particularly in suspension crystallization. This may lead to a difficulty in separation of a product from the precipitate. For example, when the easily polymerizable compound is a (meth)acrylic acid, maleic acid and manganese in the easily polymerizable compound-containing solution form a complex in crystallization, and the formed manganese maleate precipitates together with (meth)acrylic acid crystals, which are a product. This may lead to a difficulty in separation of the product from the precipitate. Here, impurities such as maleic acid are contained in an easily polymerizable compound-containing gas used for producing an easily polymerizable compound by a gas-phase reaction, and manganese is derived from a manganese-based polymerization inhibitor. Both of these are difficult to reduce to very low levels. The present inventors have found that such a precipitate causes problems. For example, a precipitate causes clogging of a liquid-discharging nozzle or line, mixing of a precipitate into a product causes delay or inhibition of polymerization, or a precipitate accumulates in a tank where the residue is received.

Here, as a result of intensive studies, the present inventors have focused on a method for producing an easily polymerizable compound, the method including: mixing an easily polymerizable compound and a manganese-based polymerization inhibitor; adjusting a manganese concentration in an easily polymerizable compound-containing solution obtained through the mixing by bringing at least a portion of the easily polymerizable compound-containing solution into contact with a cation exchange resin; and crystallizing from an easily polymerizable compound-containing solution having a manganese concentration of 5 mass ppm or lower obtained through the adjusting. The present inventors have found that such a production method is capable of sufficiently preventing formation of a precipitate of impurities and manganese derived from a manganese-based polymerization inhibitor under crystallization conditions and capable of efficiently providing a high-quality product, solving the above problems. Thereby, the present invention is achieved.

That is, the present invention relates to a method for producing an easily polymerizable compound, the method including: mixing an easily polymerizable compound and a manganese-based polymerization inhibitor; adjusting a manganese concentration in an easily polymerizable compound-containing solution obtained through the mixing by bringing at least a portion of the easily polymerizable compound-containing solution into contact with a cation exchange resin; and crystallizing from an easily polymerizable compound-containing solution having a manganese concentration of 5 mass ppm or lower obtained through the adjusting.

### - Advantageous Effects of Invention

The production method of the present invention can efficiently provide a high-quality product.

### BRIEF DESCRIPTION OF DRAWINGS

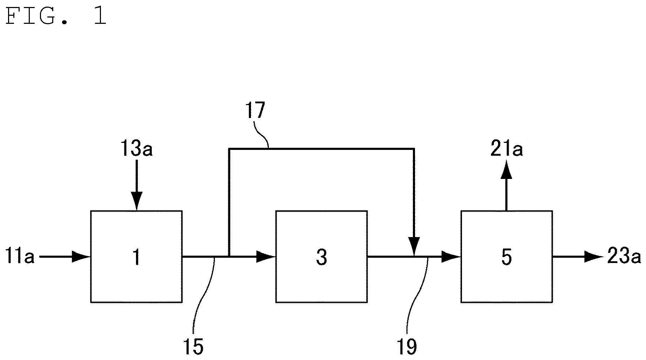

FIG. 1 is a schematic diagram of an exemplary production method of the present invention.

### DESCRIPTION OF EMBODIMENTS

The present invention is described in detail below.

It should be noted that combinations of two or more of the preferred features of the present invention described below are also preferred embodiments of the present invention.

The following first describes a method for producing an easily polymerizable compound of the present invention, followed by descriptions of a method for purifying an easily polymerizable compound of the present invention and a purification apparatus of the present invention in this order.

### (Method for producing easily polymerizable compound of the present invention)

The present invention relates to a method for producing an easily polymerizable compound, the method including: mixing an easily polymerizable compound and a manganese-based polymerization inhibitor; adjusting a manganese concentration in an easily polymerizable compound-containing solution obtained through the mixing by bringing at least a portion of the easily polymerizable compound-containing solution into contact with a cation exchange resin; and crystallizing from an easily polymerizable compound-containing solution having a manganese concentration of 5 mass ppm or lower obtained through the adjusting.

In the production method of the present invention, the mixing, the adjusting, and the crystallizing are basically performed in this order on a material to be purified. The following first describes the crystallizing, followed by descriptions of the mixing, the adjusting, and other steps in this order. In the case of continuous purification, the steps are typically performed simultaneously when the purification apparatus is viewed as a whole.

Herein, the term "easily polymerizable compound" refers to an easily polymerizable compound obtainable by the production method of the present invention, and does not refer to raw materials, by-products, and solvents in the production method of the present invention. The term "easily polymerizable compound" may also be referred to as a "target compound" or a "target". Herein, the term "impurities" refers to components other than the "easily polymerizable compound", such as raw materials, by-products, and solvents.

### <Crystallizing from an easily polymerizable compound-containing solution having a manganese concentration of 5 mass ppm or lower obtained through the adjusting>

The crystallizing includes crystallizing from an easily polymerizable compound-containing solution having a manganese concentration of 5 mass ppm or lower obtained through the adjusting, which is described below.

With a manganese concentration of 5 mass ppm or lower, the concentration of the complex of manganese and impurities is equal to or lower than its saturation solubility under the conditions of crystallizing the easily polymerizable compound. Thereby, precipitation of the complex can be sufficiently prevented and a product can be easily separated. The crystallizing is typically performed at low temperatures (e.g., in the case of a (meth)acrylic acid, the temperature of the solution or mother liquor is about 0°C to 12°C). Thus, even when the manganese concentration is low as described above, the risk of occurring polymerization of the easily polymerizable compound is low.

The easily polymerizable compound-containing solution to be fed to the crystallizing preferably has a manganese concentration of 5 mass ppm or lower, more preferably 3 mass ppm or lower, particularly preferably 2 mass ppm or lower.

In the production method of the present invention, the easily polymerizable compound-containing solution to be fed to the crystallizing preferably has a manganese concentration of 0.2 mass ppm or higher. Thereby, polymerization of the easily polymerizable compound can be further prevented in the crystallizing and in subsequent steps, particularly in recovering the easily polymerizable compound from the mother liquor or residue separated in the crystallizing. The manganese concentration is more preferably 0.3 mass ppm or higher, still more preferably 0.4 mass ppm or higher, particularly preferably 0.5 mass ppm or higher.

The manganese concentration is measured by the method described in the EXAMPLES.

The easily polymerizable compound-containing solution to be fed to the crystallizing refers to the easily polymerizable compound-containing solution having a manganese concentration of 5 mass ppm or lower obtained through the adjusting, and also refers to an easily polymerizable compound-containing solution to be fed to an apparatus used in the crystallizing (e.g., a batch-type crystallization tank or a continuous apparatus including a crystallization tank, a ripening tank, and a wash column).

The temperature in the crystallizing may be appropriately adjusted according to the type of the easily polymerizable compound to be purified. The temperature is approximately preferably -1°C to -15°C, more preferably - 1.5°C to -13.5°C, still more preferably -3.5°C to -12.5°C, particularly preferably -5°C to -11.5°C from the melting point of the pure substance. When the easily polymerizable compound to be purified is a (meth)acrylic acid, the temperature is preferably 0°C to 12°C, more preferably 1°C to 10°C, still more preferably 2°C to 8.5°C.

The temperature in the crystallizing is the temperature of the easily polymerizable compound-containing solution to be subjected to the crystallizing or the temperature of the mother liquor or residue in the easily polymerizable compound crystal-containing slurry to be subjected to the crystallizing.

The pressure condition in the crystallizing may be increased pressure, atmospheric pressure, or reduced pressure.

The crystallizing is not limited as long as it forms easily polymerizable compound crystals. The crystallizing may be continuous crystallization or batch crystallization. When the crystallizing involves formation of a slurry containing crystals and a mother liquor, as in the case of typical continuous crystallization, the mother liquor is to be separated by the crystallizing. When the crystallizing involves separation of crystals and the residue from each other and formation of a melt of the crystals, as in the case of batch crystallization using a falling film crystallizer or the like, the residue is separated by the crystallizing.

In a preferred embodiment of the production method of the present invention, the crystallizing is suspension crystallization. In suspension crystallization, manganese maleate remarkably precipitates. The production method of the present invention can sufficiently prevent such precipitation of manganese maleate.

The crystallizing can be performed using a crystallization tank and/or a ripening tank, which are described later.

The crystallizing can be suitably performed by feeding the easily polymerizable compound-containing solution having a manganese concentration of 5 mass ppm or lower obtained through the adjusting into the crystallization tank or the ripening tank.

When the crystallizing is continuous crystallization, the retention time of the easily polymerizable compound in the crystallization tank or the ripening tank used in the crystallizing may be appropriately adjusted according to the type of the easily polymerizable compound to be purified. From the viewpoint of the yield of the easily polymerizable compound obtained after purification, the efficiency of purification, and equipment investment cost, the retention time is approximately 0.02 to 6 hours. When the easily polymerizable compound to be purified is a (meth)acrylic acid, the retention time is preferably 0.05 to 5 hours, more preferably 0.1 to 4.5 hours. When the first to N-th tanks are connected in series, the retention time in the N-th tank is preferably 0.5 to 6 hours to adjust the particle size distribution of the slurry. When the compound is a (meth)acrylic acid, the retention time is more preferably 1 to 5 hours, still more preferably 1.2 to 4.5 hours.

When the crystallization tank is of a type of cooling the contents of the tank outside the tank, the retention time of the easily polymerizable compound in the crystallization tank herein refers to the sum of the retention times in the tank and in the cooling mechanism outside the tank. The retention time in each tank is calculated by dividing the sum of the volumes of the tank and the cooling mechanism outside the tank by the sum of the flow volume of the slurry fed from the tank to an upstream tank or wash column (which is present in the subsequent stage) and the flow volume of the mother liquor sent from the tank to a downstream tank or discharged from the tank to the outside of the purification apparatus.

When the easily polymerizable compound-containing solution is fed to the crystallization tank or the ripening tank, the feed rate is not limited and is, for example, 0.2 × 10³ to 4.0 × 10⁵ kg/h in the case of an industrial-scale crystallization tank or ripening tank.

The purification yield in the crystallizing is preferably 50% to 70%. The purification yield refers to the mass percentage of the product obtained in the crystallizing to the total amount of the easily polymerizable compound-containing solution fed to the crystallizing.

Through the crystallizing, manganese is concentrated depending on the purification efficiency and is contained in the mother liquor or residue. According to the production method of the present invention, as described above, the manganese concentration in the easily polymerizable compound-containing solution to be fed to the crystallizing is adjusted in advance, whereby the precipitation of the complex can be sufficiently prevented. When the purification yield falls within the above-described preferred range and the manganese concentration in the easily polymerizable compound-containing solution to be fed to the crystallizing is 0.2 to 5 mass ppm, the manganese concentration in the mother liquor or residue recovered in the crystallizing becomes about 0.7 to 10 mass ppm.

An example of the easily polymerizable compound-containing solution fed to the tank is an aqueous solution of the easily polymerizable compound. The easily polymerizable compound-containing solution typically contains impurities other than the easily polymerizable compound and water.

In the production method of the present invention, the easily polymerizable compound-containing solution to be fed to the crystallizing preferably has a purity (mass percentage) of the easily polymerizable compound of 96% by mass or lower.

The mass percentage of the easily polymerizable compound in the solution is preferably 60% by mass or higher.

In the crystallizing, the slurry containing easily polymerizable compound crystals obtained in the crystallizing may be discharged from a tank (e.g., when the purification apparatus of the present invention includes a plurality of tanks connected in series, the tank arranged last in series) and fed to the wash column.

In the crystallizing, the slurry containing easily polymerizable compound crystals may be agitated in the ripening tank.

When the slurry containing easily polymerizable compound crystals is agitated in the ripening tank, it is typically agitated using an agitator provided in the ripening tank.

The rotation speed of the agitator is preferably in the range of 5 to 500 rpm, more preferably in the range of 10 to 300 rpm.

The agitating may be intermittent, preferably basically continuous during use of the ripening tank.

When the residue formed in the crystallizing is received in a tank, the easily polymerizable compound solution containing manganese may be retained to cause precipitation of manganese maleate in the tank. This precipitate may deposit in the tank or cause clogging of a line. In response to these problems, the residue may be passed through an ion exchange resin so as to reduce the manganese concentration. The residue may be passed through an ion exchange resin by any technique. For example, an ion-exchange resin may be placed between the crystallizing section and the tank for receiving the residue, or an ion-exchange resin may be placed in the circulation line of the tank.

### <Mixing easily polymerizable compound and manganese-based polymerization inhibitor>

The mixing includes mixing an easily polymerizable compound and a manganese-based polymerization inhibitor.

The expression "mixing an easily polymerizable compound and a manganese-based polymerization inhibitor" is not limited as long as the easily polymerizable compound and the manganese-based polymerization inhibitor are mixed. For example, a manganese-based polymerization inhibitor may be added to an easily polymerizable compound, an easily polymerizable compound may be added to a manganese-based polymerization inhibitor, or an easily polymerizable compound and a manganese-based polymerization inhibitor may be introduced in one vessel and mixed. The easily polymerizable compound and the manganese-based polymerization inhibitor to be mixed may each be in the form of gas, solution, or solid.

The mixing may include mixing an easily polymerizable compound and a manganese-based polymerization inhibitor in an absorption column, for example, the manganese-based polymerization inhibitor may be directly introduced from the middle of the tower in the absorption column, or the easily polymerizable compound-containing gas may be absorbed in a liquid (e.g., water) containing a manganese-based polymerization inhibitor. Alternatively, after an easily polymerizable compound-containing solution is obtained in an absorption column and before the easily polymerizable compound-containing solution is purified in a low-boiling separation column, a manganese-based polymerization inhibitor may be added to the easily polymerizable compound-containing solution, or may be directly introduced into the column from the middle thereof. Alternatively, an easily polymerizable compound and a manganese-based polymerization inhibitor may be mixed in an absorption column, and a manganese-based polymerization inhibitor may also be added to a low-boiling separation column.

Of these, preferred is mixing an easily polymerizable compound and a manganese-based polymerization inhibitor in an absorption column. The manganese-based polymerization inhibitor may be any one containing manganese. Examples thereof include manganese dialkyldithiocarbamate, manganese diphenyldithiocarbamate, manganese formate, manganese acetate, manganese octanoate, and manganese naphthenate.

In the mixing, the mass percentage of the manganese-based polymerization inhibitor to be mixed with the easily polymerizable compound is preferably, in terms of manganese concentration, 0.1 to 100 mass ppm, more preferably 0.5 to 90 mass ppm, still more preferably 1 to 80 mass ppm, relative to the easily polymerizable compound.

In the production method of the present invention, the easily polymerizable compound is preferably an easily polymerizable compound having a reactive double bond.

In particular, in the production method of the present invention, the easily polymerizable compound is more preferably an unsaturated carboxylic acid, still more preferably a (meth)acrylic acid, particularly preferably acrylic acid. Herein, the term " (meth)acrylic acid" refers to acrylic acid and/or methacrylic acid.

The easily polymerizable compound-containing solution is not limited to one synthesized in-house, and may be one procured from outside sources.

### <Adjusting manganese concentration in easily polymerizable compound-containing solution obtained through mixing by bringing at least portion of easily polymerizable compound-containing solution into contact with cation exchange resin>

In the adjusting, the manganese concentration in an easily polymerizable compound-containing solution obtained through the mixing is reduced by bringing at least a portion of the easily polymerizable compound-containing solution into contact with a cation exchange resin. The manganese concentration in the easily polymerizable compound-containing solution can be adjusted more advantageously in terms of energy by use of a cation exchange resin compared to distillation or other techniques.

The bringing at least a portion of the easily polymerizable compound-containing solution into contact with a cation exchange resin may be performed by a continuous technique or a batch technique, for example. Preferably, the easily polymerizable compound-containing solution is passed through a cation exchange resin by a continuous technique or the like. Non-limiting examples of a resin tank include a fixed bed tank and a fluidized bed tank. The used cation exchange resin can also be reused by treating it with a strong acid solution such as sulfuric acid, nitric acid, or hydrochloric acid.

The flow rate of the easily polymerizable compound-containing solution can be selected as appropriate. The flow rate in terms of a space velocity (SV) is preferably 0.01 h⁻¹ or higher, more preferably 0.05 h⁻¹ or higher, still more preferably 0.1 h⁻¹ or higher. The flow rate is preferably 10 h⁻¹ or lower, more preferably 5 h⁻¹ or lower, still more preferably 1 h⁻¹ or lower.

The bringing at least a portion of the easily polymerizable compound-containing solution into contact with a cation exchange resin may be performed at any temperature and may be performed at a temperature within the range from the melting point to the boiling point of the easily polymerizable compound. When the easily polymerizable compound is a (meth)acrylic acid, the temperature may be within the range from 15°C to 50°C, for example.

The bringing at least a portion of the easily polymerizable compound-containing solution into contact with a cation exchange resin may be performed under increased pressure, atmospheric pressure, or reduced pressure.

In the adjusting, the easily polymerizable compound-containing solution to be brought into contact with a cation exchange resin preferably has a water concentration of 0.3 to 4% by mass. In other words, preferably, in the production method of the present invention, the easily polymerizable compound-containing solution to be fed to the adjusting has a water concentration of 0.3 to 4% by mass. When the water concentration is 0.3% by mass or higher, manganese can be removed more efficiently by the cation exchange resin. When the water concentration is 4% by mass or lower, the purification before the crystallization can be omitted or the purification in the crystallization can be easily performed.

In the adjusting, a portion of the easily polymerizable compound-containing solution may be brought into contact with a cation exchange resin, or the entire easily polymerizable compound-containing solution may be brought into contact with a cation exchange resin. Preferably, a portion of the easily polymerizable compound-containing solution is brought into contact with a cation exchange resin. Thereby, the manganese concentration in the easily polymerizable compound-containing solution to be fed to the crystallizing can be easily adjusted. Also, since not the entire easily polymerizable compound-containing solution but only a portion thereof is brought into contact with a cation exchange resin, the life of the cation exchange resin can be extended and the apparatus can be downsized, advantageously.

For example, preferably, in the production method of the present invention, the adjusting includes bringing a portion of the easily polymerizable compound-containing solution obtained through the mixing into contact with a cation exchange resin; and mixing the portion of the easily polymerizable compound-containing solution having been brought into contact with the cation exchange resin with another portion of the easily polymerizable compound-containing solution obtained through the mixing and not having been brought into contact with a cation exchange resin.

In the mixing with the easily polymerizable compound-containing solution having been brought into contact with the cation exchange resin, the mixing ratio between the portion of the easily-polymerizable compound-containing solution having been brought into contact with a cation exchange resin and the another portion of the easily-polymerizable compound-containing solution not having been brought into contact with a cation exchange resin may be adjusted as appropriate so that the manganese concentration in the easily polymerizable compound-containing solution to be subjected to the crystallizing achieves the predetermined concentration described above.

In the mixing with the easily polymerizable compound-containing solution having been brought into contact with a cation exchange resin, a purification apparatus including a bypass line, which is described later, can be suitably used, for example. To adjust the mass percentage of the easily polymerizable compound-containing solution to be brought into contact with a cation exchange resin, a distribution mechanism such as a valve or a flowmeter (a valve attached to a bypass line and/or a line for sending the easily polymerizable compound-containing solution to a column filled with a cation exchange resin or the like) may be used.

Examples of the cation exchange resin include a strongly acidic cation exchange resin having a group such as a sulfonic acid (salt) group and a weakly acidic cation exchange resin having a group such as a carboxylic acid (salt) group. One or more of these may be used. Preferred is a strongly acidic cation exchange resin, for example.

The cation exchange resin may be a gel-type cation exchange resin or a porous-type cation exchange resin, with the latter being more preferred.

### <Recovering easily polymerizable compound from mother liquor or residue separated in crystallizing>

The production method of the present invention preferably further includes recovering the easily polymerizable compound from a mother liquor or residue separated in the crystallizing.

The mother liquor or residue containing concentrated impurities used in the recovering typically contain an easily polymerizable compound (easily polymerizable compound-containing solution). The easily polymerizable compound recovered in the recovering can be reused. The recovered easily polymerizable compound can be used for another process or the like. For example, the recovered easily polymerizable compound may be fed to an apparatus in the preceding stage for reuse. Thereby, loss of the easily polymerizable compound can be reduced.

For example, the recovering is preferably distillation, but is not limited thereto. The distillation may be one for removing low-boiling components, high-boiling components such as dimers of easily polymerizable compounds, or both.

In the distillation, the separated mother liquor or residue is typically heated. In the production method of the present invention, the amount of manganese removed is adjusted in the adjusting, and thus, a trace amount of a manganese-based polymerization inhibitor remains in the separated mother liquor or residue. Thereby, polymerization can be sufficiently prevented, and additional feed of a polymerization inhibitor can be omitted or reduced in the recovering.

In the distillation, the temperature and time for heating the separated mother liquor or residue can be appropriately set depending on the purpose of distillation, the boiling points of the easily polymerizable compound and impurities in the mother liquor or residue, and the like.

The distillation may be performed under increased pressure, atmospheric pressure, or reduced pressure.

In the production method of the present invention, the easily polymerizable compound-containing solution is preferably a (meth)acrylic acid aqueous solution or a crude (meth)acrylic acid solution.

The (meth)acrylic acid aqueous solution refers to a solution in which a (meth)acrylic acid is dissolved in water. The crude (meth)acrylic acid solution refers to a solution consisting of a (meth)acrylic acid and impurities such as by-products produced during the production of the (meth)acrylic acid.

Examples of the impurities include acids such as propionic acid, acetic acid, maleic acid, benzoic acid, and acrylic acid dimers; aldehydes such as acrolein, furfural, formaldehyde, and glyoxal; acetone; methyl isobutyl ketone; toluene; and protoanemonin.

Impurities in the easily polymerizable compound-containing solution can be sufficiently removed by the production method of the present invention.

### <Obtaining easily polymerizable compound-containing solution from raw material>

The production method of the present invention preferably includes obtaining an easily polymerizable compound-containing solution from a raw material.

The obtaining an easily polymerizable compound-containing solution from a raw material preferably includes: obtaining an easily polymerizable compound-containing gas from a raw material; and obtaining an easily polymerizable compound-containing solution from the easily polymerizable compound-containing gas.

The obtaining an easily polymerizable compound-containing gas from a raw material is not limited as long as it can provide an easily polymerizable compound-containing gas. When the easily polymerizable compound is a (meth)acrylic acid, this step can be suitably performed by synthesis (catalytic vapor phase oxidation reaction) of acrylic acid described in JP 2007-182437 A (Patent Literature 1), for example.

The obtaining an easily polymerizable compound-containing solution from the easily polymerizable compound-containing gas is not limited as long as it can provide an easily polymerizable compound-containing solution. When the easily polymerizable compound is a (meth)acrylic acid, this step can be suitably performed by collecting acrylic acid described in Patent Literature 1, for example. The obtaining an easily polymerizable compound-containing solution from the easily polymerizable compound-containing gas may be performed simultaneously with the above-described mixing an easily polymerizable compound and a manganese-based polymerization inhibitor.

In the production method of the present invention, preferably, the (meth)acrylic acid is produced from at least one raw material selected from the group consisting of propane, propylene, acrolein, isobutene, methacrolein, acetic acid, lactic acid, isopropanol, 1,3-propanediol, glycerol, and 3-hydroxypropionic acid. The (meth)acrylic acid and/or raw material may also be bio-based (meth)acrylic acids derived from renewable raw materials.

In the obtaining an easily polymerizable compound-containing gas, impurities such as by-products are basically generated. For example, when the easily polymerizable compound is a (meth)acrylic acid, the impurities generated include water; acids such as propionic acid, acetic acid, maleic acid, benzoic acid, and acrylic acid dimers; aldehydes such as acrolein, furfural, formaldehyde, and glyoxal; acetone; methyl isobutyl ketone; toluene; and protoanemonin. Such impurities can be separated with excellent efficiency by purification such as the crystallizing in the production method of the present invention. Thereby, a product can be efficiently obtained.

FIG. 1 is a schematic diagram of an exemplary production method of the present invention.

An easily polymerizable compound-containing gas 11a is fed to an absorption column 1. Into the absorption column 1 is introduced absorption water 13a containing a manganese-based polymerization inhibitor for absorbing the easily polymerizable compound-containing gas 11a. The absorption water 13a absorbs the easily polymerizable compound-containing gas 11a to be an easily polymerizable compound-containing solution (aqueous solution). The solution is sent from the absorption column to the resin tank through a line 15 for sending the easily polymerizable compound-containing solution. In the middle of the line 15, an intermediate tank, a distillation apparatus (e.g., a low-boiling separation column), a filtration apparatus, and the like may be further installed. The easily polymerizable compound-containing solution sent from the absorption column to the resin tank corresponds to the easily polymerizable compound-containing solution obtained through the mixing in the present invention. The easily polymerizable compound-containing solution obtained through the mixing encompasses an easily polymerizable compound-containing solution obtained by the mixing and an easily polymerizable compound-containing solution obtained by purifying (e.g., by distillation such as low-boiling distillation or by filtration) the solution obtained by the mixing. As described above, before the distillation such as low-boiling distillation, the manganese-based polymerization inhibitor may be mixed in advance. When the easily polymerizable compound is a (meth)acrylic acid, low-boiling components such as acrolein, acetic acid, and water are removed by low-boiling distillation.

A portion of the easily polymerizable compound-containing solution obtained through the mixing is sent to a resin tank 3 through a line for sending the easily polymerizable compound-containing solution to the resin tank. The rest of the easily polymerizable compound-containing solution is passed through a bypass line 17 and mixed with the easily polymerizable compound having been passed through the resin tank 3. The combined easily polymerizable compound-containing solution has a manganese concentration adjusted to 5 mass ppm or lower, and corresponds to the easily polymerizable compound-containing solution having a manganese concentration of 5 mass ppm or lower obtained through the adjusting in the present invention. The easily polymerizable compound-containing solution having an adjusted manganese concentration to be fed to the crystallizing encompasses an easily polymerizable compound-containing solution obtained by the adjusting and an easily polymerizable compound-containing solution obtained by purifying (e.g., by distillation or by filtration) the easily polymerizable compound-containing solution obtained by the adjusting. The easily polymerizable compound-containing solution having a manganese concentration of 5 mass ppm or lower is passed through a line 19 for sending the easily polymerizable compound-containing solution from the resin tank to the crystallization tank or the ripening tank, and is fed to a crystallizing section 5 (e.g., crystallizing performed in a batch crystallization tank or a continuous crystallization tank, ripening tank, or wash column) for crystallization. In the middle of the line 19, an intermediate tank, a distillation apparatus (e.g., a low-boiling separation column), a filtration apparatus, and the like may be further installed. In the crystallizing, formation of a precipitate derived from the manganese-based polymerization inhibitor is sufficiently prevented. As a result, a high-quality easily polymerizable compound (product) 21a can be efficiently obtained from the crystallizing section 5, and a mother liquor or residue 23a is suitably recovered and recycled.

### (Method for purifying easily polymerizable compound)

The present invention also relates to a method for purifying an easily polymerizable compound, the method including: mixing the easily polymerizable compound and a manganese-based polymerization inhibitor; adjusting a manganese concentration in an easily polymerizable compound-containing solution obtained through the mixing by bringing at least a portion of the easily polymerizable compound-containing solution into contact with a cation exchange resin; and crystallizing from an easily polymerizable compound-containing solution having a manganese concentration of 5 mass ppm or lower obtained through the adjusting.

The purification method of the present invention enables efficient purification to provide a high-quality easily polymerizable compound. Preferred embodiments of the purification method of the present invention are the same as the preferred embodiments of the production method of the present invention described above.

### (Purification apparatus of the present invention)

The present invention also relates to an apparatus for purifying an easily polymerizable compound, the apparatus including: a resin tank for bringing an easily polymerizable compound-containing solution into contact with a cation exchange resin; at least one of a crystallization tank capable of forming easily polymerizable compound crystals or a ripening tank for growing easily polymerizable compound crystals; and a line for sending the easily polymerizable compound-containing solution from the resin tank to at least one of the crystallization tank or the ripening tank.

The resin tank is not limited as long as it is used to bring the easily polymerizable compound-containing solution into contact with a cation exchange resin. Preferably, the resin tank is one that allows the easily polymerizable compound-containing solution to pass through a cation exchange resin. For example, the resin tank is one filled with a cation exchange resin.

The cation exchange resin is suitably any of the cation exchange resins listed above.

The resin tank may have any size. For example, the resin tank preferably has an inner diameter of 100 to 5000 mm. The resin tank preferably has a height of 200 to 10000 mm.

The resin tank preferably has a filling capacity of a cation exchange resin of 10 to 100000 L.

The crystallization tank may include a cooling mechanism and is not limited as long as it cools the easily polymerizable compound-containing solution for precipitation of crystals, whereby a slurry containing crystals and a mother liquor can be formed or crystals and the residue can be separated from each other and a melt of the crystals can be formed. Examples of the crystallization tank include: continuous crystallization tanks such as a cooling disk crystallizer (CDC), a scraped surface cooling heat exchanger, a double-propeller (DP) crystallizer, a Kureha continuous crystal purifier (KCP), and a melt crystallization purification facility equipped with a scraped surface crystallizer and a purification column; and batch crystallization tanks such as a falling film crystallizer and a static crystallizer.

In the case of purification of an easily polymerizable compound such as an (meth)acrylic acid, which is required to be produced at high productivity, the crystallization tank is preferably of a type that the contents of the tank are cooled outside the tank. In such a case where the tank is connected to a cooling mechanism with a pipe, a portion of the easily polymerizable compound-containing solution (or crystal-containing slurry) in the tank is sent to the cooling mechanism to form crystals in the cooling mechanism, and a slurry containing the formed crystals is returned to the tank, the heat transfer area can be easily increased by increasing the number of cooling mechanisms and the crystallization tank can be easily scaled up.

The cooling mechanism in this case is not limited as long as it can cool the easily polymerizable compound solution to precipitate crystals. Preferred examples of the cooling mechanism include a cooling disk crystallizer (CDC) and a scraped surface cooling heat exchanger, each of which can have a large heat transfer area.

The cooling disk crystallizer is not limited as long as it cools the easily polymerizable compound-containing solution to precipitate crystals and scrapes off the precipitated crystals. The cooling disk crystallizer may be, for example, one including a cylinder and cooling plates that separate the inside of the cylinder and having a structure in which crystals are formed on the wall surfaces of the cooling plates and an agitator blade with a wiper is rotated in the cylinder to scrape off the crystals.

The scraped surface cooling heat exchanger is not limited as long as it cools the easily polymerizable compound-containing solution to precipitate crystals and scrapes off the precipitated crystals. The scraped surface cooling crystallizer may be, for example, one including a double pipe and having a structure in which a cooling medium is passed through the outer pipe and the easily polymerizable compound solution (or crystal-containing slurry) in the tank is passed through the inner pipe to form crystals on the wall surface of the inner pipe, and a shaft having a scraping blade is rotated in the inner pipe to scrape off the crystals.

The crystallization temperature in the crystallization tank may be the temperature in the crystallizing described above. When the temperature in the crystallization tank is high, high-purity crystals are formed, but in the case of using a scraped surface cooling heat exchanger in the crystallization tank, for example, scraping off the crystals in the crystallization tank may require a large amount of power or other problems may occur. When the temperature difference between the cooling medium and the inside of the crystallization tank is too large, problems may occur such as blocking of the scraper during use of the scraped surface cooling heat exchanger in the crystallization tank, for example. This may lead to a difficulty in continuous operation. Thus, when the temperature in the crystallization tank is high, the temperature difference between the cooling medium and the inside of the crystallization tank is required to be reduced and the amount of crystals formed per unit heat transfer area is required to be reduced. When the temperature in the crystallization tank is low, low-purity crystals are formed, but in the case of using a scraped surface cooling heat exchanger in the crystallization tank, the crystallization tank requires only a small amount of power for scraping off crystals. Thereby, the scraper is less likely to be blocked even when the temperature difference between the cooling medium and the inside of the crystallization tank is large. As a result, the temperature difference between the cooling medium and the inside of the crystallization tank may be increased to increase the amount of crystals formed per unit heat transfer area. However, when the crystallization temperature is too low, crystals with smaller particle sizes, which are less likely to settle, tend to be formed.

The purification apparatus of the present invention includes the crystallization tank and/or the ripening tank. The purification apparatus of the present invention may include one or multiple tanks. When the purification apparatus of the present invention includes multiple tanks (first to N-th tanks where the first tank is the most downstream tank and the N-th tank is the most upstream tank), these tanks are preferably connected in series. In this case, the purification apparatus of the present invention typically includes a line for sending the slurry containing easily polymerizable compound crystals from one tank to another tank optionally via a solid-liquid separator. In this case, the purification apparatus of the present invention typically further includes a line for feeding a liquid to be purified containing an easily polymerizable compound or a slurry containing easily polymerizable compound crystals to at least one tank. When the purification apparatus of the present invention further includes a ripening tank, the ripening tank preferably corresponds to a tank that sends a liquid to the wash column, that is, the N-th tank.

The ripening tank is not limited as long as it can keep the easily polymerizable compound crystals suspended in the tank. When the crystals are kept for a certain period of time, fine crystals are melted by Ostwald ripening and large crystals grow further, so that the crystal size distribution becomes narrow. As a result, high-quality crystals can be obtained, and by subjecting such crystals to the subsequent purification in the wash column or the like, the purification efficiency in the wash column can be further improved.

The temperature in the ripening tank may be the temperature in the above-described crystallizing.

The ripening tank typically includes in the vicinity of the bottom a discharge port for discharging the slurry containing easily polymerizable compound crystals from the ripening tank.

The ripening tank may include a baffle in the tank.

Examples of a material of the baffle include metals such as stainless steel and resins.

The ripening tank may include multiple baffles.

The ripening tank may further include in the vicinity of the top roof a discharge port for discharging the mother liquor in a supernatant part from the ripening tank. The discharged mother liquor can be recycled. Thereby, the yield of the easily polymerizable compound can be improved. For example, the discharged mother liquor can be returned to the tank in the previous step (preceding stage). The nozzle or pipe that constitutes the discharge port may be made of any material. Examples of the material include metals such as stainless steel and alloys.

The ripening tank may include one or multiple mother-liquor discharge ports.

The ripening tank may include a mechanism (e.g., partition plate, weir) to prevent the easily polymerizable compound crystals from entering the mother-liquor discharge port. Such mechanisms can further prevent crystals from entering the mother-liquor discharge port.

The crystallization tank or the ripening tank may have any size. For example, each tank preferably has an inner diameter of 100 to 50000 mm. Each tank preferably has a height of 1000 to 100000 mm.

Instruments such as a thermometer, a pressure gauge, a liquid level gauge (e.g., of radar type), and a level switch (e.g., of float type) may be provided in the main body or periphery of the crystallization tank or the ripening tank. The ripening tank may include a sight glass (an observation window) in its side wall or the like. The sight glass may be covered with a cover. The ripening tank may include a hole such as a manhole or a hand hole (a hole for inserting a hand for maintenance) in its top roof, side wall, or the like. The ripening tank may include a rupture device or the like in its top roof or the like. The

### numbers of these are not limited.

Preferably, the purification apparatus of the present invention further includes: a bypass line for passing at least a portion of the easily polymerizable compound-containing solution therethrough without contacting the portion with a cation exchange resin; and a mixing section for mixing the portion of the easily polymerizable compound-containing solution that has been passed through the bypass line and another portion of the easily polymerizable compound-containing solution that has been brought into contact with a cation exchange resin.

The mixing section is not limited as long as it is configured to mix a portion of the easily polymerizable compound-containing solution that has been passed through the bypass line and another portion of the easily polymerizable compound-containing solution that has been brought into contact with a cation exchange resin. For example, the mixing section may be simply a T-shaped line or a mixing tank.

The bypass line is typically branched from a line for sending the easily polymerizable compound-containing solution to the resin tank and is connected to a line for sending the easily polymerizable compound-containing solution from the resin tank to the crystallization tank and/or the ripening tank.

In the purification apparatus of the present invention, preferably, the line for sending the easily polymerizable compound-containing solution to the resin tank and/or the bypass line includes a distribution mechanism that controls the flow rate.

Examples of the distribution mechanism include a valve and a flow meter which are each attached to the line for sending the easily polymerizable compound-containing solution to the resin tank and/or the bypass line. Further, a valve (e.g., a three-way valve) for switching a flow path may be installed at the T-junction between the line for sending the easily polymerizable compound-containing solution to the resin tank and the bypass line. When a valve for switching a flow path is installed at the T-junction, another valve or the like may or may not be attached to the line for sending the easily polymerizable compound-containing solution to the resin tank and/or the bypass line.

Preferably, the purification apparatus of the present invention further includes: an absorption column for collecting an easily polymerizable compound-containing gas to obtain an easily polymerizable compound-containing solution; and a line for sending the easily polymerizable compound-containing solution from the absorption column to the resin tank.

The absorption column allows the easily polymerizable compound-containing gas which is the reaction product obtained in a reactor to be absorbed into water in which a polymerization inhibitor is dissolved, thereby providing the easily polymerizable compound-containing solution.

In the middle of the line for sending the easily polymerizable compound-containing solution from the absorption column to the resin tank, an intermediate tank, a low-boiling distillation apparatus, a filtration apparatus, and the like may be installed. As described above, when the easily polymerizable compound is a
(meth)acrylic acid, low-boiling components such as acrolein, acetic acid, and water are removed by low-boiling distillation.

The purification apparatus of the present invention is preferably one capable of performing continuous purifying. For example, the purification apparatus may further include a wash column (preferably a wash column that forcibly transports crystals) as a subsequent stage of the tank in the present invention.

When the purification apparatus of the present invention further includes the wash column, the purification apparatus of the present invention may include a line for feeding the easily polymerizable compound crystal-containing slurry from a tank (e.g., when the purification apparatus of the present invention includes multiple tanks connected in series, the tank arranged last in series) in the purification apparatus of the present invention to the wash column instead of a line for sending out the easily polymerizable compound crystal-containing slurry from the tank as a product.

The purification apparatus of the present invention may further include a line for sending out the product from the wash column.

The purification apparatus of the present invention may further include a line for returning the mother liquor from a tank or apparatus in a subsequent stage to a tank or apparatus in a preceding stage.

The purification apparatus of the present invention may further include a mechanism that controls the amount of the slurry to be sent and the amount of the mother liquor to be returned. Examples of the control mechanism include valves attached to the lines.

The purification apparatus of the present invention may appropriately include different apparatuses commonly used in purification apparatuses.

### EXAMPLES

The present invention will be described in more detail below with reference to examples, but the present invention is not limited by the following examples, and appropriate modifications may be made within the scope that can conform to the gist of the above and later descriptions. All of them are included in the technical scope of the present invention.

Unless otherwise specified, "%" indicates "% by mass" and "part(s)" indicates "part(s) by mass."

### (Method for preparing acrylic acid aqueous solution)

An acrylic acid aqueous solution was prepared according to the method described in WO 2010/032665 as follows: propylene was catalytically oxidized in vapor phase to obtain an acrylic acid-containing gas; and the acrylic acid-containing gas was brought into contact with water containing manganese acetate added as a polymerization inhibitor in an absorption column.

The acrylic acid aqueous solution obtained in the absorption column had a manganese concentration of 10 mass ppm and a water concentration of 4 % by mass.

### (Resin tank)

A resin tank was an apparatus having the following dimensions.
Inner diameter: 400 mm
Height: 800 mm
Filling capacity of cation exchange resin: 50 L

The cation exchange resin used in the resin tank was DIAION PK208 available from Mitsubishi Chemical Corporation.

### (Crystallizing)

The acrylic acid aqueous solution was fed at 20 kg/h to a scraped surface crystallizer with a jacket containing a cooling medium, and the crystal concentration was adjusted to 15% to obtain an acrylic acid slurry. Operation was performed where the slurry was fed to a hydraulic wash column at 110 kg/h, and the crystals were melted in a heat exchanger, whereby the purification yield reached 50%.

### (Recovering)

A portion of the mother liquor discharged from the wash column in the crystallizing was fed at 10 kg/h to a distillation column. The distillation column was operated at an operating pressure of 100 hPa and an operating temperature of 100°C.

### (Analysis of manganese concentration)

The acrylic acid aqueous solution obtained in the absorption column, the acrylic acid aqueous solution immediately before being fed to the crystallizing, and the product (acrylic acid) were sampled; each sample was diluted 10 times with a 0.1% by weight nitric acid aqueous solution; and the dilutions were subjected to measurement of manganese concentration using an ICP emission spectrophotometer (Thermo Scientific, iCAP6000 SERIES).

### <Example 1>

The acrylic acid aqueous solution obtained in the absorption column was fed to a resin tank (25°C) filled with the above-described cation exchange resin. To adjust the manganese concentration in the acrylic acid aqueous solution to be fed to the crystallizing, a bypass line was provided configured to prevent at least a portion of the acrylic acid aqueous solution obtained in the absorption column from flowing through the resin tank. The flow rate of the acrylic acid aqueous solution was adjusted so that a portion of the solution was passed through the resin tank at 10 kg/h and another portion of the solution was passed through the bypass line at 10 kg/h. Thereafter, the portions of the acrylic acid aqueous solution flowing through the tank and the bypass line were combined and fed to the crystallizing. The manganese concentration in the acrylic acid aqueous solution immediately before being fed to the crystallizing was 5 mass ppm.

As a result, no manganese maleate was precipitated during the crystallizing, and no manganese was detected in the obtained product (acrylic acid). Line clogging due to precipitation of manganese maleate and the like did not occur, and no additional feed of a polymerization inhibitor was required in the mother-liquor recovery. Thereby, a stable working was achieved. Table 1 shows the results.

### <Example 2>

Operation was performed as in Example 1, except that the flow rate of the acrylic acid aqueous solution was adjusted so that a portion of the solution was passed through the resin tank at 19.6 kg/h and another portion of the solution was passed through the bypass line at 0.4 kg/h. The manganese concentration in the acrylic acid aqueous solution immediately before being fed to the crystallizing was 0.2 mass ppm.

As a result, no manganese maleate was precipitated during the crystallizing, and no manganese was detected in the obtained product (acrylic acid). Line clogging due to precipitation of manganese maleate and the like did not occur, and no additional addition of a polymerization inhibitor was required in the mother-liquor recovery. Thereby, a stable working was achieved. Table 1 shows the results.

### <Comparative Example 1>

Operation was performed as in Example 1, except that the acrylic acid aqueous solution was not fed to the resin tank but was fed only to the bypass line. Since the acrylic acid aqueous solution immediately before being fed to the crystallizing was not passed through the resin tank, it was the same as the acrylic acid aqueous solution obtained in the absorption column, and had a manganese concentration of 10 mass ppm.

As a result, manganese maleate was precipitated in the crystallizing, and a product (acrylic acid) containing manganese was obtained, which had poor quality. Thereafter, the working was continued. However, manganese maleate caused line clogging, leading to a need for stopping of the working. Table 1 shows the results.

### <Comparative Example 2>

Operation was performed as in Example 1, except that the flow rate of the acrylic acid aqueous solution was adjusted so that a portion of the solution was passed through the resin tank at 8 kg/h and another portion of the solution was passed through the bypass line at 12 kg/h. The manganese concentration in the acrylic acid aqueous solution immediately before being fed to the crystallizing was 6 mass ppm.

As a result, manganese maleate was precipitated in the crystallizing, and a product (acrylic acid) containing manganese was obtained, which had poor quality. Thereafter, the working was continued. No clogging occurred unlike Comparative Example 1, but adhesion of manganese maleate to the line was observed. Thus, the working was somehow continued by performing an operation accompanying an increase in pressure, for example. Table 1 shows the results.

### <Example 3>

Operation was performed as in Example 1, except that the acrylic acid aqueous solution was not fed to the bypass line but was fed only to the resin tank. The manganese concentration in the acrylic acid aqueous solution immediately before being fed to the crystallizing was 0 mass ppm.

As a result, no manganese maleate was precipitated during the crystallizing, and no manganese was detected in the obtained product (acrylic acid). Although line clogging due to precipitation of manganese maleate and the like did not occur, polymerization occurred in the mother-liquor recovery. Thus, the working was temporarily stopped, and an additional polymerization inhibitor was added to restart the working. Table 1 shows the results.

**[Table 1]**

| | Manganese concentration (ppm) immediately before being fed to crystallizing | Precipitation of manganese maleate in crystallizing |
|---|---|---|
| Comparative Example 1 | 10 | Occurred |
| Comparative Example 2 | 6 | Occurred |
| Example 1 | 5 | Not occurred |
| Example 2 | 0.2 | Not occurred |
| Example 3 | 0 | Not occurred |

The following describes the critical significance of the numerical range in the present invention understood through the examples and comparative examples described above. Specifically, it was found that when the easily polymerizable compound-containing solution having a manganese concentration of 5 mass ppm or lower was subjected to crystallization, an advantageous effect of efficiently obtaining a high-quality product can be remarkably achieved.

In the examples and comparative examples, an acrylic acid aqueous solution was used as the easily polymerizable compound-containing solution. In the purifying in the production of an easily polymerizable compound, when a manganese-based polymerization inhibitor is mixed for the purpose of preventing polymerization, and the easily polymerizable compound-containing solution (especially a (meth)acrylic acid solution or a crude (meth)acrylic acid solution) containing the manganese-based polymerization inhibitor is subjected to crystallization, impurities and manganese in the easily polymerizable compound-containing solution form a complex as a precipitate under crystallization conditions, causing a difficulty in separation of a product from the precipitate. As for easily polymerizable compound-containing solutions, such a problem occurs in the same mechanism. Thus, when the easily polymerizable compound-containing solution having a manganese concentration of 5 mass ppm or lower is subjected to crystallization, the advantageous effects of the present invention are definitely exhibited. At least when a (meth)acrylic acid aqueous solution or crude (meth)acrylic acid solution having a manganese concentration of 5 mass ppm or lower is subjected to crystallization, the advantageous effects of the present invention can be sufficiently demonstrated by the examples and comparative examples, and the technical significance of the present invention is supported.

### REFERENCE SIGNS LIST

1: absorption column
3: resin tank
5: crystallizing section
11a: easily polymerizable compound-containing gas
13a: absorption water
15: line (for sending easily polymerizable compound-containing solution from absorption column to resin tank)
17: bypass line
19: line (for sending easily polymerizable compound-containing solution from resin tank to crystallization tank or ripening tank)
21a: easily polymerizable compound (product)
23a: mother liquor or residue

## Claims

1. A method for producing an easily polymerizable compound, the method comprising:
mixing an easily polymerizable compound and a manganese-based polymerization inhibitor;
adjusting a manganese concentration in an easily polymerizable compound-containing solution obtained through the mixing by bringing at least a portion of the easily polymerizable compound-containing solution into contact with a cation exchange resin; and
crystallizing from an easily polymerizable compound-containing solution having a manganese concentration of 5 mass ppm or lower obtained through the adjusting.

2. The method for producing an easily polymerizable compound according to claim 1,
wherein the easily polymerizable compound-containing solution to be fed to the adjusting has a water concentration of 0.3 to 4% by mass.

3. The method for producing an easily polymerizable compound according to claim 1 or 2,
wherein the easily polymerizable compound-containing solution to be fed to the crystallizing has a manganese concentration of 0.2 mass ppm or higher.

4. The method for producing an easily polymerizable compound according to any one of claims 1 to 3,
wherein the adjusting comprises:
bringing a portion of the easily polymerizable compound-containing solution obtained through the mixing into contact with a cation exchange resin; and
mixing the portion of the easily polymerizable compound-containing solution having been brought into contact with the cation exchange resin with another portion of the easily polymerizable compound-containing solution obtained through the mixing and not having been brought into contact with a cation exchange resin.

5. The method for producing an easily polymerizable compound according to any one of claims 1 to 4, the method further comprising recovering the easily polymerizable compound from a mother liquor or residue separated in the crystallizing.

6. The method for producing an easily polymerizable compound according to any one of claims 1 to 5,
wherein the easily polymerizable compound-containing solution is a (meth)acrylic acid aqueous solution or a crude (meth)acrylic acid solution.

7. The method for producing an easily polymerizable compound according to any one of claims 1 to 6, the method comprising:
obtaining an easily polymerizable compound-containing gas from a raw material; and
obtaining an easily polymerizable compound-containing solution from the easily polymerizable compound-containing gas.

8. The method for producing an easily polymerizable compound according to any one of claims 1 to 7,
wherein the easily polymerizable compound is a (meth)acrylic acid.

9. The method for producing an easily polymerizable compound according to claim 8,
wherein the (meth)acrylic acid is produced from at least one raw material selected from the group consisting of propane, propylene, acrolein, isobutene, methacrolein, acetic acid, lactic acid, isopropanol, 1,3-propanediol, glycerol, and 3-hydroxypropionic acid.

10. A method for purifying an easily polymerizable compound, the method comprising:
mixing the easily polymerizable compound and a manganese-based polymerization inhibitor;
adjusting a manganese concentration in an easily polymerizable compound-containing solution obtained through the mixing by bringing at least a portion of the easily polymerizable compound-containing solution into contact with a cation exchange resin; and
crystallizing from an easily polymerizable compound-containing solution having a manganese concentration of 5 mass ppm or lower obtained through the adjusting.

11. An apparatus for purifying an easily polymerizable compound, the apparatus comprising:
a resin tank for bringing an easily polymerizable compound-containing solution into contact with a cation exchange resin;
at least one of a crystallization tank capable of forming easily polymerizable compound crystals or a ripening tank for growing easily polymerizable compound crystals; and
a line for sending the easily polymerizable compound-containing solution from the resin tank to at least one of the crystallization tank or the ripening tank.

12. The apparatus for purifying an easily polymerizable compound according to claim 11, the apparatus further comprising:
a bypass line for passing at least a portion of the easily polymerizable compound-containing solution therethrough without contacting the portion with a cation exchange resin; and
a mixing section for mixing the portion of the easily polymerizable compound-containing solution that has been passed through the bypass line and another portion of the easily polymerizable compound-containing solution that has been brought into contact with a cation exchange resin.

13. The apparatus for purifying an easily polymerizable compound according to claim 11 or 12, the apparatus further comprising:
an absorption column for collecting an easily polymerizable compound-containing gas to obtain an easily polymerizable compound-containing solution; and
a line for sending the easily polymerizable compound-containing solution from the absorption column to the resin tank.
